# EUROPEAN PATENT APPLICATION

(11) **EP 1 580 557 A1**
(43) Date of publication of application: **28.09.2005**
(21) Application number: 03774159.2
(22) Date of filing: 21.11.2003
(51) Int. Cl.: G01N 33/569, G01N 33/543

(54) **METHOD OF EXAMINING STAPHYLOCOCCUS AUREUS**

(30) Priority: 22.11.2002 JP 2002339526
(71) Applicant: DAIICHI PURE CHEMICALS CO., LTD., Tokyo 103-0027 (JP)
(72) Inventor: Horii, Toshinobu, Nagoya-shi, Aichi 464-0858 (JP); Kondo, Akira, Daiichi Pure Chemicals Co., Ltd., Ryugasaki-shi, Ibaraki 301-0852 (JP); Kanno, Takashi, Hamamatsu-shi, Shizuoka 431-3125 (JP); Maekawa, Masato, Hamamatsu-shi, Shizuoka 431-3125 (JP)
(74) Representative: Hartz, Nikolai
(86) International application number: PCT/JP2003/014946
(87) International publication number: WO 2004/048975

(57) **Abstract**

This invention relates to a method for testing *S*. *aureus* in a specimen by an immunoassay using an antibody against protein A wherein at least one mouse IgG1 monoclonal antibody is used in the immunoassay, and a test kit for testing S. *aureus* in a specimen wherein the kit at least comprises a mouse IgG1 anti-protein A monoclonal antibody and a reagent for detecting the labeled protein A.

Use the present invention enables detection of *S. aureus* in the sample in a short time and at a high sensitivity.

## Description

### Technical Field

This invention relates to a method for testing *Staphylococcus aureus* in a specimen at a high sensitivity without cultivating the *Staphylococcus aureus.*

### Background ART

*Staphylococcus aureus (S. aureus)* is a bacterium of *Staphylococcus spp.* which is gram-positive and which usually appears under the microscope as irregular grape-like clusters, and it is also a major pathogenic bacterium which produces various toxins and enzymes that induce purulent diseases in human and mammals. *S. aureus* is also a typical bacterial strain which causes food poisoning in human. MRSA (methicillin-resistant *S. aureus)* is also a species of *S. aureus,* and it has attracted special attention since it is a causal bacterium of nosocomial infection.

A popular procedure that has often been used in detecting *S. aureus* is a procedure wherein pure cultivation is conducted in a selective isolation medium, and suspicious colonies are chosen for further identification tests of the bacterial species, and the identification has been conducted, for example, by checking production of coagulase, or by immunologically measuring the antibody against thermonuclease, enterotoxin, or protein A produced by the *S. aureus* (Archivfur Lebensmittelhygiene, 35, 97 (1984); and Japanese Patent Application Laid-Open Nos. H06-68824 and H09-211000).

The conventional test methods, however, all required cultivation of the bacteria, and a substantial time was needed before the identification of the bacterium. When the results of the identification were obtained, patients were often too serious to utilize the test results. In addition, there has been a fair possibility that the bacterium present in a minute amount in blood is left undetected in patients receiving antibacterials.

Recently, several highly sensitive measurements were reported which do not require precultivation of the specimen, and these measurements use protein A for the target (Journal of Immunological Methods, 83 (1985) 169-177; Journal of Immunological Methods, 117 (1989) 83-89; and Journal of Immunological Methods, 142 (1991) 53-59). These measurements all employ sandwich enzyme immunoassay, and respectively use the combination of immobilized rabbit anti-protein A antibody and rabbit anti-protein A antibody labeled with alkaline phosphatase, the combination of immobilized rabbit anti-protein A antibody and F (ab') 2 of rabbit anti-protein A antibody labeled with HRP, and the combination of chicken anti-protein A antibody (IgY) and biotinylated rabbit anti-protein A antibody.

However, protein A is known to have a site which binds with the Fcdomainof immunoglobulin (IgG) frommammals, and accordingly, the immunoassays as described above had the problem of interaction of the IgG used in both or either one of the immobilized antibody and the labeled antibody with the site of the protein A that binds to the Fc domain.

Another attempt that has also been made for quick, high sensitivity detection of bacterial infection is gene amplification (Kato., I., Protein, Nucleic Acid and Enzyme, Vol. 35, page 2957 (1990)) . Since *S. aureus is* an indigenous bacterium, its detection is associated with the risk shared by indigenous bacteria that those not infected are also detected as positive by the minute contamination of the bacterial gene. With regard to the MRSA, a method utilizing detection of *mecA* which is a gene involved in the antibiotic resistance of MRSA has been recently developed, and this method has enabled MRSA identification with no need to wait the results of the sensitivity test by utilizing gene amplification (Ubukata et al., J. Clin. Microbiol., 30, pages 1728-1733 (1992)), and this method is already in clinical use. This *mecA* gene, however, is sometimes found in *Staphylococcus spp.* such as CNS other than S. *aureus,* and detection of *mecA* gene has been associated with the difficulty of clearly differentiating between MRSA and CNS.

### Disclosure of the Invention

An object of the present invention is to provide a method which is capable of detecting S. *aureus* in a specimen in a short time and at a high sensitivity.

In view of the situation as described above, the inventors of the present invention have made an intensive investigation on immunoassays using the antibody against protein A, and found that an immunoassay of high sensitivity using at least one mouse IgG1 monoclonal antibody is capable of detecting or quantitatively determining the protein A antigen in the blood, urine, sputum, spinal fluid, pleural effusion, ascites, pus, or other body fluid components from patients infected by S. *aureus* without being affected by IgG, and that S. *aureus* in a specimen can be tested in a short time by using such method. The present invention has been completed on such a finding.

Accordingly, the present invention provides a method for testing S. *aureus* in a specimen by an immunoassay using an antibody against protein A wherein at least one mouse IgG1 monoclonal antibody is used in the immunoassay.

The present invention also provides a test kit for testing *S. aureus* in a specimen wherein the kit at least comprises mouse IgG1 anti-protein A monoclonal antibody and a reagent for detecting the labeled protein A.

The present invention has enabled to test protein A at a high sensitivity without being affected by the IgG and the like in the specimen, and to conveniently and quickly test whether or not the patient is infected by *S*. *aureus.* The present invention can also determine the infection even if the S. *aureus* were MRSA.

### Brief Description of the Drawings

FIG. 1 is a view showing the standard curve of purified protein A measured by sandwich enzyme immunoassay 1.
FIG. 2 is a view showing the standard curve of the purified protein A measured by sandwich enzyme immunoassay 2.
FIG. 3 is a view showing amount of the protein A in the supernatant that has been measured over time.
FIG. 4 is a view showing measurements of the protein A in serum (3 specimens) and urine (3 specimens) of healthy donors, and serum (4 specimens) and urine (2 specimens) of the patients suffering from S. *Aureus* infections.

### Best Mode for Carrying Out the Invention

The method for testing S. *aureus* of the present invention is a method wherein protein A produced by S. *aureus* is directly detected in the specimen by an immunoassay.

Accordingly, the present invention does not require precultivation of the specimen (test specimen) or increase of the concentration of the protein A in the specimen by centrifugation, column separation, precipitation, or the like.

In the present invention, the term *"S. aureus"* includs methicillin-resistant *S*. *aureus.*

The specimen used in the present invention is not limited as long as there is possibility that S. *aureus* is present, and exemplary specimens include blood, urine, sputum, spinal fluid, pleural effusion, ascites, pus, and other body fluid components. More specifically, the present invention is capable of directly detecting or quantitatively determining the protein A without precultivation of the test specimen or increase in the concentration of the protein A of the test specimen not only when the specimen are those containing the bacteria at a high concentration such as sputum or urine but also when the specimen are those containing the bacteria at a low concentration (for example, 10 to 10³ cfu/mL) such as blood, pleural effusion, or ascites.

In addition to the use of the specimen with no further dilution, the specimen may also be used after diluting with a buffer such as phosphate buffer. The buffer used in the dilution of the specimen may simultaneously contain an adequate protein such as .BSA, HSA, gelatin, or the like for stabilization of the antigen.

The immunoassay used in the present invention is not particularly limited as long as it is an immunoassay of high sensitivity which can measure the protein A produced by *S*. *aureus* to the order of 100 pg/mL or less, and more preferably 10 pg/mL or less, and exemplary immunoassays include enzyme immunoassay, radioimmmunoassay, immunochromatography, fluorescent immunoassay, and other immunoassays known in the art. The preferred, however, is an enzyme immunoassay in view of the safety and the sensitivity.

An enzyme immunoassay is an immunoassay wherein amount of the relevant antibody or antigen is measured by detecting enzyme activity of the enzyme labeled antibody using an enzyme such as peroxidase, alkaline phosphatase, or β-galactosidase. Enzyme immunoassays using various enzyme detection means are known such as colorimetric enzyme immunoassay, fluorescent enzyme immunoassay, and chemiluminescent enzyme immunoassay (CLEIA). In the colorimetric enzyme immunoassay, enzyme activity is quantitatively determined by measuring coloring of the chromogenicsubstancegenerated by decomposition of the enzymatic substrate such as hydrogen peroxide/o-phenylenediamine, p-nitrophenylphosphate, oro-nitrophenyl-β-D-galactopyranoside by the enzyme. In the fluorescent enzyme immunoassay, fluorescence is generated by decomposition of the fluorescent substrate such as 4-hydroxyphenyl acetic acid, 4-methyl-umbelliferyl phosphate, or 4-methyl-umbelliferyl-β-galactoside by the catalytic activity of the enzyme, and the fluorescence intensity is measured to quantitatively determine the enzyme activity. In the chemiluminescent enzyme immunoassay (CLEIA), chemiluminescent substance is excited by the catalytic activity of the enzyme, and amount of the light emitted when it returns to the ground state is measured to quantitatively determine the enzyme activity. Among these, use of a chemiluminescent enzyme immunoassay is particularly preferable in the present invention since no light source is required for the chemiluminescence of the chemiluminescent substance, and detection at a sensitivity higher than the colorimetric or the fluorescent immunoassays is possible.

Exemplary enzymes used in the CLEIA include peroxidase, alkaline phosphatase, β-galactosidase, glucose oxidase, and glucose-6-phosphodehydrogenase, and exemplary chemiluminescent substances include luminols such as luminol derivatives and isoluminol derivatives, lucigenin (N,N'-dimethyl-9, 9'-bisacridinium nitrate), and bis(2,4,6-trichlorophenyl)oxalate. Among these, the preferred is the combination of luminol /hydrogen peroxide for the substrate with peroxidase (Tsuji, A., Protein, Nucleic Acid and Enzyme, Special Edition, vol. 31, pages 51-63 (1987)). If necessary, D-(-)-luciferin, 6-hydroxybenzothiazole, p-iodophenol, or the like may be simultaneously incorporated in the chemiluminescent system to enhance the chemiluminescence.

The immunoassay of the present invention is not particularly limited for its measurement procedure, and the assay may be conducted either by a competitive assay or a sandwich assay. However, use of a double antibody sandwich assay is preferable in view of conducing the assay at a high sensitivity.

In the immunoassay of the present invention, at least one mouse IgG 1 monoclonal antibody is used for the antibody against protein A, and this antibody may be used either for the primary antibody or the secondary antibody.

Mouse IgG 1 monoclonal antibody is a monoclonal antibody obtained from mouse which belongs to antibody subclass IgG 1, and it has low affinity for the site in the protein A that binds to Fc domain of immunoglobulin. Use of this antibody enables reliable detection of the protein A in the specimen simultaneously containing a substance which non-immunologically binds to an antibody from a mammal (a substance having the site which binds to Fc of the immunoglobulin) or a microorganism producing such an antibody, or in the specimen simultaneously containing IgG, and a highly sensitive and specific testing of the S. *aureus* is thereby enabled.

When a dual antibody immunoassay is employed, at least one of the primary antibody and the secondary antibody used may comprise the mouse IgG 1 monoclonal antibody which recognizes a non-Fc-binding site of the protein A. The other one of the primary antibody and the secondary antibody may preferably comprise a mouse IgG 1 monoclonal antibody which recognizes a site different from the mouse IgG 1 monoclonal antibody as described above; an antibody fragment (such as F(ab')2, Fab', or Fab fragment) obtained from a polyclonal antibody IgG of a mammal other than mouse (for example, rabbit or goat) by treating with an enzyme such as pepsin or by reducing treatment to cleave off the Fc site; an antibody fragments (F (ab')2, Fab' , Fab fragment, and the like) obtained by cleaving off Fc site from a monoclonal antibody other than IgG 1, which recognizes non-Fc binding site of the protein A; or an antibodywhich does not react with Fc-binding site of protein A, such as an IgG antibody from rat, IgM antibody from a mammal, chicken IgY antibody; and in particular, use of a F(ab')2 fragment obtained by treating polyclonal antibody IgG from a mammal with pepsin is preferable.

Selection of such an antibody enables to completely omit the interaction between IgG and the Fc binding site of the protein A.

The mouse IgG 1 monoclonal antibody may be obtained, for example, according to the procedure normally used in the art by preparing a mouse monoclonal antibody which recognizes protein A, and selecting a monoclonal antibody whose subclass is IgG 1 using subclass typing kit for mouse IgG.

The anti-protein A polyclonal antibody and the monoclonal antibody can also be prepared by a method known in the art using a purified antigen.

Next, an embodiment of sandwich assay using the mouse IgG 1 monoclonal antibody for the primary antibody is described.
1) Mouse IgG1 anti-protein A monoclonal antibody is immobilized on an adequate solid carrier by adsorption.
2) The antibody is brought in contact with the specimen containing the *S. aureus* for specific bonding of the protein A produced by the S. *aureus* in the specimen with the antibody immobilized on the solid carrier to thereby immobilize the protein A in the specimen to the solid carrier.
3) The carrier having protein A immobilized thereon is brought in contact with a solution containing a labeled antibody against the protein A (secondary antibody) to bind the secondary antibody to the solid carrier through the immobilized anti-protein A antibody and the protein A.
4) The thus immobilized secondary antibody is measured by means of the label of the secondary antibody to detect or quantitatively determine the protein A in the specimen.

Non-limiting examples of the solid carrier used for immobilizing the primary antibody include glass or plastic tube, plate, well, or beads, magnetic particles, latex, membrane, and fibers.

The immobilization can be accomplished by physical adsorption, or alternatively, by chemical bonding using a crosslinking agent such as glutaraldehyde or carbodiimide. For example, immobilization may be conducted by contacting the antibody diluted with phosphate buffer with the surface of the solid carrier, and incubating the carrier at 37°C for 60 minutes.

The labeling of the secondary antibody may be accomplished by direct labeling with a radioisotope, an enzyme, biotin, a fluorescent substance, a chemiluminescent substance, gold colloid, latex, ferrite particle, or the like. The secondary antibody, however, is preferably labeled with an enzyme as described above in view of high safety and expectation for good measurement results. Exemplary preferable enzymes used for the labeling include peroxidase, alkaline phosphatase, and galactosidase which are highly stable and whose enzyme activity can be readily determined.

The secondary antibody bonded can be detected or quantitatively determined by any method known in the art, for example, by directly detecting or quantitatively determining the secondary antibody itself that has been labeled with an enzyme, a luminescent substance, a fluorescent substance, or the like, or by using a tertiary antibody which specifically binds to the secondary antibody and labeling this tertiary antibody by various methods and detecting or quantitatively determining the label of the tertiary antibody.

The secondary antibody is preferably detected by reacting the secondary antibody labeled with an enzyme with a substrate (chromogenic substrate, fluorescent substrate, or chemiluminescent substrate) which is specific for the enzyme, and detecting the signal associated with the color development, fluorescence, luminescence, or the like induced by the reaction with a measuring device. Use of the chemiluminescent substrate which enables high sensitivity detection is particularly preferable.

The test kit for infections caused by *S. aureus of* the present invention is a kit for carrying out the test method for *S*. *aureus* in the specimen as described above, and the kit at least comprises the mouse IgG1 anti-protein A monoclonal antibody as described above, and a reagent for detecting the label such as an enzyme, and optionally, a buffer solution for diluting the specimen, a buffer solution for diluting various reagents, a washing solution, and the like.

### Examples

Next, the present invention is described in further detail by referring to the Examples which by no means limit the scope of the invention.

### Example 1

### (1) Preparation of a plate having primary antibody immobilized thereto

Mouse IgG 1 anti-protein A monoclonal antibody diluted with 25mM phosphate buffer solution (PBS) containing 150mM NaCl to 10 µg/mL was dispensed in the wells of a plate at 50 µL/well, and the plate was allowed to stand overnight at 4°C for immobilization of the antibody. The content was removed from the plate the next day, and after adding 280 µL/well of BlockAce (manufactured by Dainippon Pharmaceutical) diluted to 4 folds with purified water, the plate was allowed to stand overnight or longer at 4°C. In use, the plate washed three times with PBS containing 0.05% Tween20 (PBS-T) was used for the reaction.

### (2) Preparation of biotinylated secondary antibody

500 µL (1mg/mL) of goat anti-protein A antibody F(ab')2 obtained by digesting goat anti-protein A polyclonal antibody with pepsin was dialyzed against 0 . 1M sodium hydrogen carbonate. A solution of 0.05 mg of Sulfo-NHS-LC-Biotin (manufactured by Pierce) in 50 µL of the carbonate buffer solution was added dropwise to the antibody solution with stirring. After stirring at room temperature for 4 hours, and dialyzing against PBS overnight at 4°C, the antibody solution was used as the biotinylated secondary antibody.

### Example 2

### (1) Measurement of protein A by sandwich enzyme immunoassay (1)

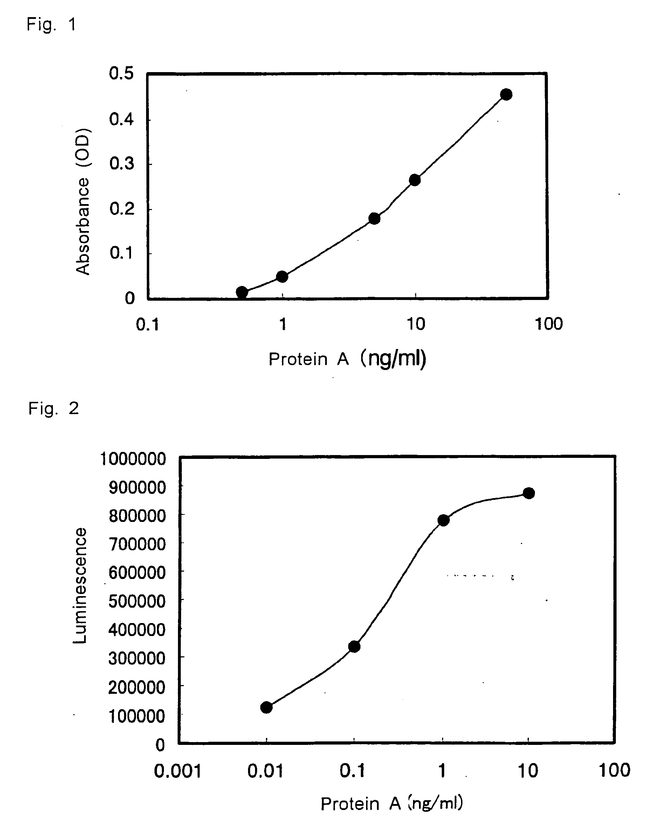

50 µL/well of purified protein A diluted with diluted BlockAce (BlockAce diluted to 10 folds with purified water) to 0. 5, 1, 5, 10, and 50 ng/mL was added to the wells of the microtiter plate having the mouse IgG 1 anti-protein A monoclonal antibody immobilized thereto produced in Example 1(1). After allowing the reaction to take place overnight at 4°C, the wells were washed with PBS-T, and 50 µL/well of biotinylated secondary antibody of Example 1 diluted with diluted BlockAce to 2 . 9 µg/mL was added. The reaction was then allowed to proceed at room temperature for 1 hour, and the wells were washed with PBS-T. In the meanwhile, avidinated HRP (manufactured by Zymed) was diluted with 0.2% BSA-PBS to 4000 folds, and 50 µL/well of this dilution was added to the well, and the reaction was allowed to proceed at room temperature for 1 hour. After washing, 50 µL/well of 0. 1M citrate buffer solution, containing 2 mg/mL o-phenylenediamine and 5.9 mM hydrogen peroxide, pH 5 was added, and the reaction was allowed to proceed at room temperature for 30 minutes, and the reaction was ceased by adding 50 µL/well of 1.5N sulfuric acid. The measurement was conducted by using a plate reader for ELISA at a wavelength of 492 nm. The resulting standard curve is shown in FIG. 1.

The measurement was possible in the protein A concentration range of 0.5 to 50 ng/mL.

### (2) Measurement of DNase by sandwich enzyme immunoassay (2)

50 µL/well of purified protein A diluted with diluted BlockAce to 0.01, 0.1, 1.0, and 10 ng/mL was added to the wells of the white microtiter plate (manufactured by Dynex technologies) having the mouse IgG 1 anti-protein A monoclonal antibody immobilized thereto. After allowing the reaction to take place overnight at 4°C, the wells were washed with PBS-T, and 50 µL/well of biotynylated secondary antibody of Example 1 diluted with diluted BlockAce to 2.9 µg/mL was added. The reaction was then allowed to proceed at room temperature for 1 hour, and the wells were washed with PBS-T. In the meanwhile, avidinylated HRP (manufactured by Zymed) was diluted with 0.2% BSA-PBS to 4000 folds, and 50 µL/well of this dilution was added to the well, and the reaction was allowed to proceed at room temperature for 1 hour. After washing, 100 µL/well of Super Signal ELISA Femto Substrate (manufactured by Pierce) was added, and luminescence of 10 seconds was integrated with a luminometer. The resulting standard curve is shown in FIG. 2.

The measurement was possible in the protein A concentration range of 0.01 to 10 ng/mL.

### Example 3: Measurement of the amount of protein A in the culture supernatant over time

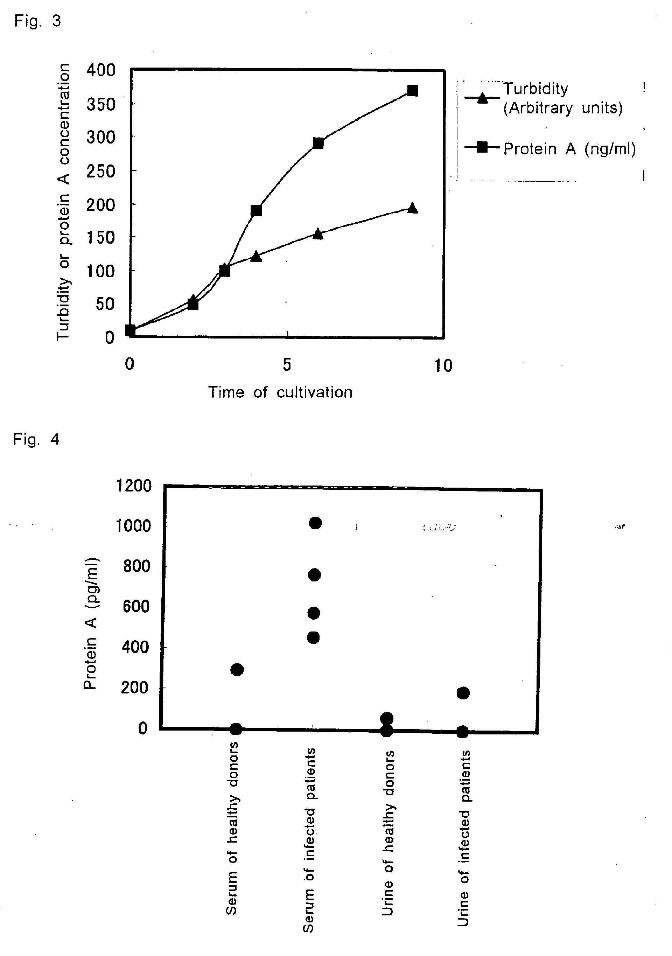

To 10 mL of heart-infusion broth was added 80 µL of overnight culture of the clinically isolated strain of S. *aureus* (MRSA1932), and the cultivation was continued. 1 mL portion of the culture was collected over time, and the collected culture was centrifuged for 2 minutes. The supernatant was filtered with 0. 2 µm membrane, and the filtrate was stored at -30°C until measurement. In order to measure the amount of the protein A secreted in accordance with the growth of the bacterium, the sample was diluted 25 to 200 folds with diluted BlockAce for measurement by ELISA. The results are shown in FIG. 3.

As demonstrated by the results, production of protein A increased corresponding to with the growth phase of the bacterium, namely, at a faster rate in the logarithmic phase compared to the stationary phase.

### Example 4: Measurement of protein A in the serum and urine of the patients

Protein A in the serum and urine of the patients suffering from S. *aureus* and the healthy donors were measured according to the procedure described in Example 2 (2). The results are shown in FIG. 4.

While no protein A was detected in the serum of the healthy donor, presence of protein A was confirmed in the serum and the urine of the infected patients.

## Claims

1. A method for testing *Staphylococcus* aureus in a specimen by an immunoassay using an antibody against protein A wherein at least one mouse IgG1 monoclonal antibody is used in the immunoassay.

2. The test method according to claim 1 wherein the immunoassay is a dual antibody sandwich enzyme immunoassay.

3. The test method according to claim 2 wherein a primary antibody and a secondary antibody are used for the immunoassay, and the mouse IgG1 monoclonal antibody is used for one of the primary and secondary antibodies, and an antibody fragment produced by cleaving off Fc domain from a polyclonal anti-protein A antibody from a mammal by an enzymatic treatment is used for the other one of the primary and the secondary antibodies.

4. The test method according to any one of claims 1 to 3 wherein the mouse IgG1 monoclonal antibody is an antibody which recognizes the protein A at a site except at the Fc-binding site.

5. The test method according to any one of claims 2 to 4 wherein the enzyme immunoassay is an enzyme chemiluminescent immunoassay.

6. The test method according to claim 5 wherein the enzyme chemiluminescent immunoassay is the one using peroxidase for the enzyme and to be labeled a luminol for the chemiluminescent substance.

7. The test method according to any one of claims 1 to 6 wherein the specimen is a body fluid component.

8. The test method according to any one of claims 1 to 7 wherein *Staphylococcus* aureus is methicillin-resistant *Staphylococcus aureus.*

9. A test kit for testing *Staphylococcus aureus in a* specimen wherein the kit at least comprises a mouse IgG1 anti-protein A monoclonal antibody and a reagent for detecting the labeledprotein A.
